# EUROPEAN PATENT APPLICATION

(11) **EP 2 008 865 A1**
(43) Date of publication of application: **31.12.2008**
(21) Application number: 07741787.1
(22) Date of filing: 17.04.2007
(51) Int. Cl.: B60N 2/24, A47C 7/74, A61F 7/08, B60N 2/44

(54) **VEHICLE-USE CREW HEATING DEVICE**

(30) Priority: 18.04.2006 JP 2006114416
(71) Applicant: Toyota Jidosha Kabushiki Kaisha, Toyota-shi, Aichi 471-8571 (JP)
(72) Inventor: HATTORI, Koji, Toyota-shi, Aichi 471-8571 (JP)
(74) Representative: Kuhnen & Wacker
(86) International application number: PCT/JP2007/058351
(87) International publication number: WO 2007/123127

(57) **Abstract**

The invention provides a more comfortable vehicle interior space by improving various symptoms of the crew. In a vehicle-use crew heating device 10, a plurality of seat heaters 36, 38, 40, and 42 are divisionally arranged in a vehicle-use seat 12 according to a plurality of symptoms (for example, excessive sensitivity to cold, stiff shoulder, lumbago, swelling foot, fatigue and the like) to be improved of a seated crew, the seat heaters 36, 38, 40 and 42 are selectively operated according to the symptoms (for example, excessive sensitivity to cold, stiff shoulder, lumbago, swelling foot, fatigue and the like) to be improved of the seated crew, and a region corresponding to the symptom to be improved of the seated crew is locally warmed up (receives thermal stimulation). Accordingly, it is possible to improve various symptoms (for example, excessive sensitivity to cold, stiff shoulder, lumbago, swelling foot, fatigue and the like) which are different according to the situation of the crew, and it is possible to provide a more comfortable vehicle interior space.

## Description

### TECHNICAL FIELD

The present invention relates to a vehicle-use crew heating device, and more particularly to a vehicle-use crew heating device which is provided with a heating element in a vehicle-use seat, and is configured such as to improve a symptom such as lumbago, a stiff shoulder or the like on the basis of a thermal stimulation of the heating element.

### BACKGROUND ART

Conventionally, as an apparatus for improving the symptom such as lumbago, stiff shoulder or the like of the crew, the following apparatus has been known (for example, refer to JP-A No. 2005-247097). For example, the JP-A No. 2005-247097 discloses an example of a vehicle-use seat with a blood circulation improving function. In this example described in the JP-A No. 2005-247097, a conductive coil and an oscillator are incorporated within a seat main body, and the structure is made such that an extremely low frequency micro ac magnetic field can be applied toward a portion in which a blood circulation injury of the seated crew is in danger of being generated, by actuating the conductive coil and the oscillator. Further, it is possible to promote an improvement of the blood circulation injury and an enhancement of the blood circulation by applying the extremely low frequency micro ac magnetic field to the region in which the blood circulation injury of the crew is generated as mentioned above, thereby preventing and recovering lumbago, stiff shoulder and the like.

Further, as the apparatus provided with the heating element in the vehicle-use seat, the following apparatus has been known (for example, refer to JP-A No. 2004-55219). For example, an example of a seat heater is disclosed in the JP-A No. 2004-55219. In the example described in the JP-A No. 2004-55219, a plurality of seat heaters are provided in the vehicle-use seat, and the structure is made such that a calorific power can be differentiated per a plurality of seat heaters. Further, the structure is made such that sufficient warmth can be quickly felt in spite of a small calorific power as a whole, by setting the calorific power of each of the seat heaters according to a position with which a human body of the crew is in contact.

### DISCLOSURE OF THE INVENTION

### PROBLEM TO BE SOLVED BY THE INVENTION

However, in spite that the symptom of the crew is different such as an excessive sensitivity to cold, stiff shoulder, lumbago, swelling foot, fatigue and the like according to an occasional situation, only the particular symptom such as lumbago, stiff shoulder or the like is always set to a target of the symptom to be improved, in the example described in the JP-A No. 2005-247097. Further, since the prevention and the recovery are aimed by directly applying a magnetic field to the particular position at which the blood circulation injury is generated, an effect can not be expected in an improvement of a general condition, a coldness of a hand or foot point to which the vehicle-use seat can not directly apply the magnetic field or the like. Further, in the example described in the JP-A No. 2004-55219, the various symptoms of the crew are not considered at all. Accordingly, there is a room of improvement for providing a more comfortable vehicle interior space by improving the various symptoms according to the situation of the crew.

The invention is made by taking the circumstances mentioned above into consideration, and an object of the invention is to provide a vehicle-use crew heating device which can provide a more comfortable vehicle interior space by improving various symptoms of the crew.

### MEANS FOR SOLVING THE PROBLEM

In order to solve the problem mentioned above, in accordance with claim 1, there is provided a vehicle-use crew heating device, wherein a plurality of heating elements are divisionally arranged at least one of a seat cushion and a seat back of a vehicle-use seat according to a plurality of symptoms to be improved of a seated crew, and the plurality of heating elements being selectively operated according to a symptom to be improved of the seated crew.

In the vehicle-use crew heating device described in claim 1, a plurality of heating elements are divisionally arranged at least in one of the seat cushion and the seat back of the vehicle-use seat according to a plurality of symptoms (for example, excessive sensitivity to cold, stiff shoulder, lumbago, swelling foot, fatigue and the like) of the seated crew that are to be improved, and a plurality of heating elements are selectively operated according to the symptoms (for example, excessive sensitivity to cold, stiff shoulder, lumbago, swelling foot, fatigue and the like) of the seated crew that are to be improved, and a local region corresponding to the symptom to be improved of the seated crew is warmed up (receives thermal stimulation). Accordingly, it is possible to improve the various symptoms (for example, excessive sensitivity to cold, stiff shoulder, lumbago, swelling foot, fatigue and the like) which are different according to the situation of the crew, and it is possible to provide a more comfortable vehicle interior space.

In accordance with claim 2, there is provided a vehicle-use crew heating device as described in claim 1, wherein a plurality of heating elements include a first heating element provided in the seat back at a region supporting a shoulder portion of the seated crew, a second heating element provided in the seat back at a region supporting a lumbar portion of the seated crew, and a third heating element provided in the seat cushion at a region supporting a thigh portion of the seated crew.

In the vehicle-use crew heating device described in claim 2, in accordance with the structure mentioned above, the first to third heating elements can be selectively operated, for example, as follows.

That is, if the symptom to be improved of the seated crew is assumed to be an excessive sensitivity to cold, the second heating element and the third heating element are selectively operated, the second heating element being provided in the seat back at the region supporting the lumbar portion of the seated crew, and the third heating element being provided in the seat cushion at the region supporting the thigh portion of the seated crew. Accordingly, since the lumbar portion, which is thought to play the role of sensing the temperature of the whole of the human body, is warmed up by the second heating element, thereby improving the function of the autonomic nerves, blood circulation to the whole body, particularly to deletions portions such as the hands and feet and the like is increased. Further, the thigh portion, which forms a pathway for blood circulation to the lower limbs, is warmed up by the third heating element; and the feet are warmed to the toes. Thereby, an excessive sensitivity to cold of the seated crew is improved.

Further, if, for example, the symptom to be improved of the seated crew is assumed to be discomfort of the shoulders, the second heating element and the first heating element are selectively operated, the second heating element being provided in the seat back at the region supporting the lumbar portion of the seated crew and the first heating element being provided in the seat back at the region supporting the shoulder portion of the seated crew. Accordingly, since the lumbar portion, which is thought to play the role of sensing the temperature of the whole of the human body, is warmed up by the second heating element, thereby improving the function of the autonomic nerves, blood circulation to the whole body is increased. Further, since the shoulder portion of the seated crew that generates stiff shoulder is warmed up by the first heating element, and blood circulation is increased, it is possible to increase the discharge of metabolic decomposition products and increase the supply of oxygen and nutrients. Further, it is possible to expect an effect that a knotted muscle is loosened up by the heat. Thereby, stiff shoulder of the seated crew is improved.

Further, if, for example, the symptom to be improved of the seated crew is assumed to be lumbago, the second heating element is selectively operated, the second heating element being provided in the seat back at the region supporting the lumbar portion of the seated crew. Accordingly, since the lumbar portion, which is thought to play,the role of sensing the temperature of the whole of the human body, is warmed up by the second heating element, thereby improving the function of the autonomic nerves, blood circulation to the whole body is increased. Further, since the lumbar portion of the seated crew that generates lumbago is warmed up by the second heating element, and blood circulation is increased, it is possible to increase the discharge of metabolic decomposition products and increase the supply of oxygen and nutrients. Further, it is possible to expect the effect that knotted muscles are loosened up by the heat. Thereby, the lumbago of the seated crew is improved.

Further, if, for example, the symptom to be improved of the seated crew is assumed to be swelling foot, the second heating element and the third heating element are selectively operated, the second heating element being provided in the seat back at the region supporting the lumbar portion of the seated crew , and the third heating element being provided in the seat cushion at the region supporting the thigh portion of the seated crew. Accordingly, since the lumbar portion, which is thought to play the role of sensing the temperature of the whole of the human body, is warmed up by the second heating element, thereby improving the function of the autonomic nerves, blood circulation to the whole body, particularly the deletions portions such as the hands and feet, is increased. Further, the thigh portion, which forms a pathway for blood circulation to the lower limbs, is warmed up by the third heating element, and the feet are warmed to the toes. Thereby, contracted muscles are loosened up, the functions of the lymph vessels are activated, and the swelling foot of the seated crew is improved.

Further, if, for example, the symptom to be improved of the seated crew is assumed to be fatigue, the first heating element, the second heating element and the third heating element are selectively operated, the first heating element being provided in the seat back at the region supporting the shoulder portion of the seated crew, the second heating element being provided in the seat back at the region supporting the lumbar portion of the seated crew and the third heating element being provided in the seat cushion at the region supporting the thigh portion of the seated crew. Accordingly, the shoulder portion, the lumbar portion and the thigh portion are respectively warmed up by the first to third heating elements. Therefore, it is possible to promote the blood circulation of approximately the whole body from the shoulder portion to the lumbar portion and the thigh portion, and to the lower limbs by extension, and the fatigue of the seated crew is improved.

In accordance with claim 3, there is provided a vehicle-use crew heating device as described in claim 1 or 2, wherein the crew heating device is provided with a crew state detecting means for detecting a state of a seated crew, and a control means for selectively actuating the plurality of heating elements based on the detection results of the crew state detecting means.

In the vehicle-use crew heating device described in claim 3, the state of the seated crew is detected by the crew state detecting means. Accordingly, the various symptoms (for example, excessive sensitivity to cold, stiff shoulder, lumbago, swelling foot, fatigue and the like) which are different according to the situation of the crew can be detected as the symptom to be improved. Further, in the vehicle-use crew heating device described in claim 3, the control means selectively actuates a plurality of heating elements based on the results of detection of the crew state detecting means mentioned above. Accordingly, a suitable region according to the symptom to be improved of the seated crew is locally warmed up (receives thermal stimulation). In accordance with the structure mentioned above, it is possible to improve the various symptoms (for example, excessive sensitivity to cold, stiff shoulder, lumbago, swelling foot, fatigue and the like) which are different according to the situation of the crew, and it is possible to provide a further more comfortable vehicle interior space.

In accordance with claim 4, there is provided a vehicle-use crew heating device as described in any one of claim 1 to 3, wherein the crew heating device is provided with an operation intent detecting means for detecting an operation intent of the seated crew, and a control means for selectively actuating the plurality heating elements based on the detection results of the operation intent detecting means.

In the vehicle-use crew heating device described in claim 4, if the operation intent of the seated crew is detected by the operation intent detecting means, the control means selectively actuates a plurality of heating elements based on the detection results of the operation intent detecting means. Accordingly, the crew expresses the operation intent (by for example, operating a switch) for improving the symptom that the crew feels at that moment, whereby the region coinciding with this operation intent is locally warmed up (receives thermal stimulation). Thereby, it is possible for the crew to improve the symptom (for example, excessive sensitivity to cold, stiff shoulder, lumbago, swelling foot, fatigue and the like) which he or she feels at that moment, by his or her own will, and it is possible to provide a more comfortable vehicle interior space.

In accordance with claim 5, there is provided a vehicle-use crew heating device as described in any one of claim 1 to 4, wherein the crew heating device is provided with a crew-sensed-temperature adjusting means capable of adjusting a temperature which the crew senses, and a control means for actuating the crew-sensed-temperature adjusting means the plurality of heating elements in an interlocking manner.

In the vehicle-use crew heating device described in claim 5, the control means actuates the crew-sensed-temperature adjusting means together with a plurality of heating elements so as to generate heat, and adjusts the temperature which the crew senses. Accordingly, it is possible, for example, to locally warm up an appropriate region coinciding with the symptom to be improved of the seated crew by a plurality of heating elements, while also maintaining the temperature within the vehicle interior space at a suitable temperature according to the season, via the crew-sensed-temperature adjusting means, or while warming up in a supplementary manner a region in the body of the seated crew which cannot be warmed up by the plurality of heating elements mentioned above; also via the crew-sensed-temperature adjusting means (that is, while maintaining the temperature sensed by the crew at an appropriate level). Thereby, the vehicle interior space becomes more comfortable.

In accordance with claim 6, there is provided a vehicle-use crew heating device as described in any one of claim 1 to 5, wherein each of the plurality of heating elements is configured such that a temperature of a center portion becomes higher than a temperature of a peripheral portion thereof.

In the vehicle-use crew heating device described in claim 6, each of a plurality of heating elements is configured such that the temperature of the center portion becomes higher than the temperature of the peripheral portion thereof. Accordingly, it is possible to set the warming up region of the body of the seated crew to within a small area, and it is possible to more locally warm up the region corresponding to the symptom (for example, excessive sensitivity to cold, stiff shoulder, lumbago, swelling foot; fatigue and the like) to be improved of the seated crew. Therefore, it is possible to improve the various symptoms (for example, excessive sensitivity to cold, stiff shoulder, lumbago, swelling foot, fatigue and the like) which are different according to the situation of the crew, without imparting a stuffy feeling to the seated crew, and it is possible to provide a more comfortable vehicle interior space.

In accordance with claim 7, there is provided a vehicle-use crew heating device as described in any one of claim 1 to 6, wherein the crew heating device is provided with a relaxation supplying means comprising any one of a massaging device capable of massaging the seated crew, a low frequency stimulation device capable of applying low frequency stimulation to the seated crew, a magnetic stimulation device capable of applying magnetic stimulation, or an aromatic incense generating device capable of supplying aromatic incense to the seated crew, or a combination thereof, and a control means actuating the relaxation supplying means the plurality of heating elements in an interlocking manner.

In the vehicle-use crew heating device described in claim 7, the control means actuates the relaxation supplying means (any one of a massaging device capable of massaging the seated crew, a low frequency stimulation device capable of applying low frequency stimulation to the seated crew, a magnetic stimulation device capable of applying magnetic stimulation, and an aromatic incense generating device capable of supplying aromatic incense to the seated crew, or a combination thereof), together with a plurality of heating elements being actuated so as to generate heat. Accordingly, in addition to the fact that the region corresponding to the symptom to be improved of the seated crew is locally warmed up (receives thermal stimulation) by a plurality of heating elements, the seated crew is relaxed by the relaxation supplying means. Thereby, it is possible to provide a comfortable vehicle interior space for the seated crew.

### EFFECT OF THE INVENTION

As in detail mentioned above, in accordance with the invention, it is possible to improve the various symptoms of the crew so as to prove the more comfortable vehicle interior space.

### BRIEF DESCRIPTION OF THE DRAWINGS

Fig. 1 is a view showing a general structure of a vehicle-use crew heating
device in accordance with a first exemplary embodiment of the invention.
Fig. 2 is a perspective view showing a structure of a vehicle-use seat provided in the vehicle-use crew heating device in accordance with the first exemplary embodiment of the invention.
Fig. 3 is an explanatory view showing a structure of a seat heater provided in the vehicle-use crew heating device in accordance with the first exemplary embodiment, and a temperature characteristic of the seat heater.
Fig. 4 is a plan view showing a structure of an operation panel provided in the vehicle-use crew heating device in accordance with the first exemplary embodiment of the invention.
Fig. 5 is a flow chart showing a motion of the vehicle-use crew heating device in accordance with the first exemplary embodiment of the invention.
Fig. 6 is a view explaining a difference of an operation between the vehicle-use crew heating device in accordance with the first exemplary embodiment of the invention and a conventional vehicle-use seat heater apparatus.
Fig. 7 is a view showing a general structure of a vehicle-use crew heating device in accordance with a second exemplary embodiment of the invention.
Fig. 8 is a view explaining a motion of the vehicle-use crew heating device in accordance with the second exemplary embodiment of the invention.
Fig. 9A is a view explaining the motion of the vehicle-use crew heating device in accordance with the second exemplary embodiment of the invention.
Fig. 9B is a view explaining the motion of the vehicle-use crew heating device in accordance with the second exemplary embodiment of the invention.
Fig. 10A is a view explaining the motion of the vehicle-use crew heating device in accordance with the second exemplary embodiment of the invention.
Fig. 10B is a view explaining the motion of the vehicle-use crew heating device in accordance with the second exemplary embodiment of the invention.
Fig. 11 is a view showing a general structure of a vehicle-use crew heating device in accordance with a third exemplary embodiment of the invention.
Fig. 12 is a view showing a general structure of a vehicle-use crew heating device in accordance with a fourth exemplary embodiment of the invention.
Fig. 13 is a perspective view showing a structure of a vehicle-use seat provided in the vehicle-use crew heating device in accordance with the fourth exemplary embodiment of the invention.
Fig. 14 is a flow chart showing a motion of the vehicle-use crew heating device in accordance with the fourth exemplary embodiment of the invention.
Fig. 15 is a view showing a general structure of a vehicle-use crew heating device in accordance with a fifth exemplary embodiment of the invention.
Fig. 16 is a view showing a modified example of the vehicle-use crew heating device in accordance with the fifth exemplary embodiment of the invention.

### DETAILED DESCRIPTION OF THE INVENTION

### [First Exemplary Embodiment]

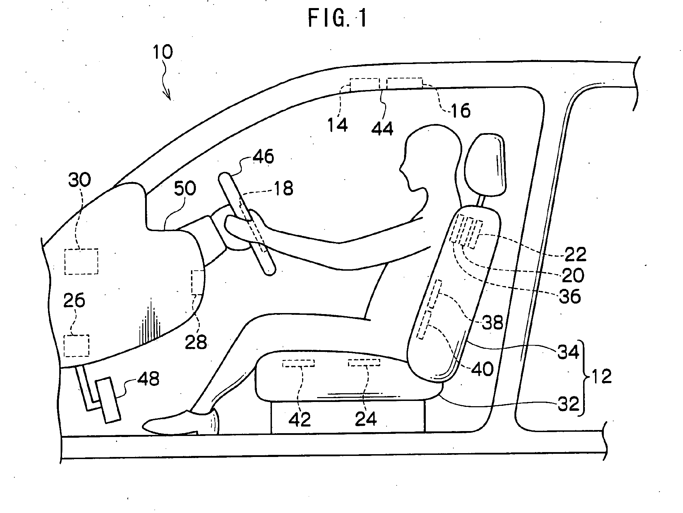

First, a description will be given of a first exemplary embodiment of the invention with reference to Figs. 1 to 6.

A vehicle-use crew heating device 10 in accordance with a first exemplary embodiment of the invention shown in Fig. 1 is preferably equipped, for example, in a vehicle such as a passenger motor vehicle or the like, and is provided with a vehicle-use seat 12, various sensors (a camera 14, an infrared ray sensor 16, a heart rate sensor 18, a blood circulation sensor 20, pressure sensitive sensors 22 and 24, and a pedal operation sensor 26) serving as a crew state detecting means, an operation panel 28 serving as an operation intent detecting means, and a control circuit 30 serving as a control means, as main components.

The vehicle-use seat 12 is arranged as a driver seat in the present exemplary embodiment, and is configured such as to have a seat cushion 32 and a seat back 34. Further, a plurality of seat heaters 36, 38, 40. and 42 serving as a heating element capable of locally warming up a body of a seated crew are divisionally arranged in the vehicle-use seat 12.

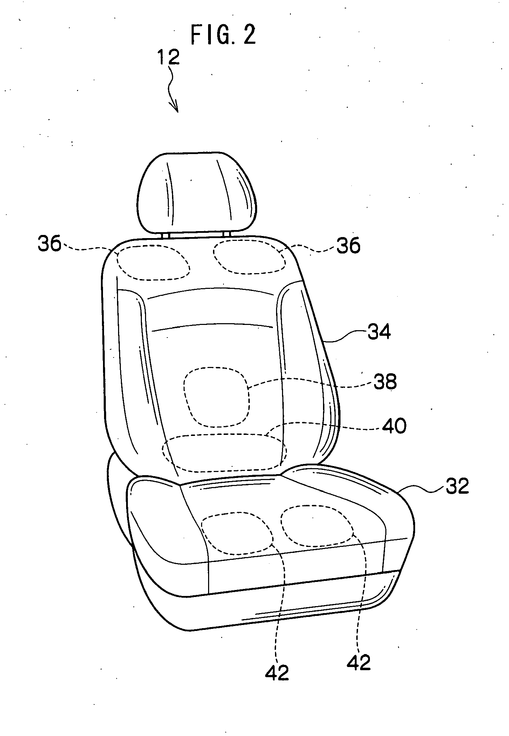

That is, as shown in Fig. 2, the seat heaters 36 serving as a first heating element are provided at a region supporting a shoulder portion of the seated crew in the seat back 34 so as to form right and left pair, the seat heater 38 and the seat heater 40 serving as a second heating element are provided at a region supporting a lumbar portion of the seated crew in the seat back 34 so as to form upper and lower pair, and the seat heaters 42 serving as a third heating element are provided at a region supporting a thigh portion of the seated crew in the seat cushion 32 so as to form right and left pair.

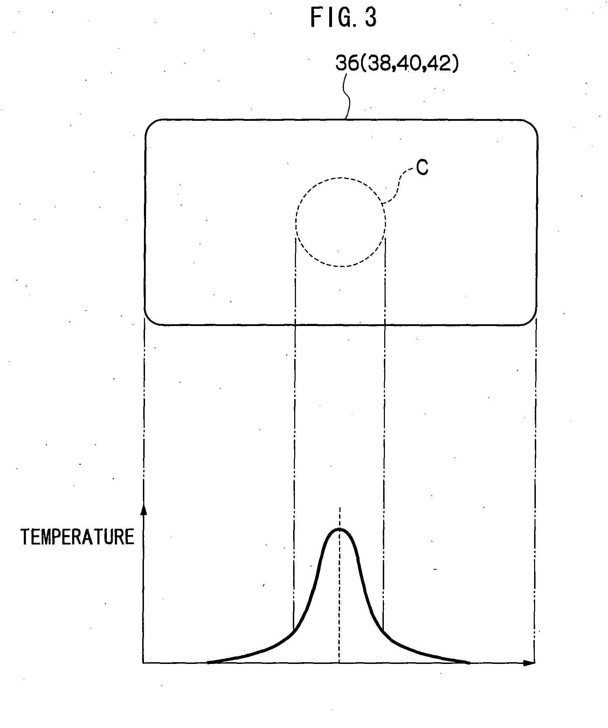

Further, each of the seat heaters 36, 38, 40 and 42 is configured by a thin plate body having a quadrangular shape in a plan view, and is configured such that a temperature of a center portion C becomes higher than a temperature of a peripheral portion thereof at a time of operating, as shown in Fig. 3.

The camera 14 in the various sensors provided in the vehicle is arranged in a ceiling portion 44 within a passenger room. The camera 14 is configured such as to photograph a posture of a whole body, a motion state of hand and foot, a facial-expression and the like of the crew seating on the vehicle-use seat 12, and output a photographed data to the control circuit 30 mentioned below.

Further, the infrared ray sensor 16 is arranged in the ceiling portion 44 within the passenger room in adjacent to the camera 14. The infrared ray sensor 16 is configured such as to detect a body temperature distribution of the crew seating on the vehicle-use seat 12 and output a data corresponding to the detected body temperature distribution to the control circuit 30 mentioned below. Further, the heart rate sensor 18 is provided integrally in a steering wheel 46. The heart rate sensor 18 is configured such as to detect a heart rate of the crew seating on the vehicle-use seat 12 and output a heart rate data to the control circuit 30 mentioned below. In this case, the heart rate sensor 18 may be provided integrally in the vehicle-use seat 12.

The blood circulation sensor 20 is arranged at a region supporting the shoulder portion of the seated crew in the seat back 34 of the vehicle-use seat 12. The blood circulation sensor 20 is configured such as to measure a blood circulation of the shoulder portion of the seated crew and output a measured data to the control circuit 30 mentioned below.

The pressure sensitive sensor 22 is arranged at a region supporting the shoulder portion of the seated crew in the seat back 34 of the vehicle-use seat 12 (a position with which the shoulder portion of the seated crew is brought into contact), in such a manner that a whole surface of the sensor is pressed by the shoulder portion of the seated crew. The pressure sensitive sensor 22 is configured such as to measure a hardness of a muscle in the shoulder portion of the seated crew, and output a measured data to the control circuit 30 mentioned below.

On the other hand, the pressure sensitive sensor 24 is arranged at a region supporting a hip portion of the seated crew in the seat cushion 32 of the vehicle-use seat 12 (a position with which the hip portion of the seated crew is brought into contact) in such a manner that a whole surface of the sensor is pressed by the hip portion of the seated crew. The pressure sensitive sensor 24 is configured such as to measure a force (a seat pressure) pressed by the hip portion of the seated crew, and output a measured data to the control circuit 30 mentioned below.

The pedal operation sensor 26 is arranged at a position near an operation pedal 48 (an accelerator pedal, a brake pedal and a clutch pedal) provided in the driver seat of the vehicle. The pedal operation sensor 26 is configured such as to detect an operation state of the operation pedal 48 (an operation state of any one or all of the accelerator pedal, the brake pedal and the clutch pedal) provided in the driver seat of the vehicle, and output a pedal operation signal to the control circuit 30 mentioned below every time when the operation pedal 48 is operated.

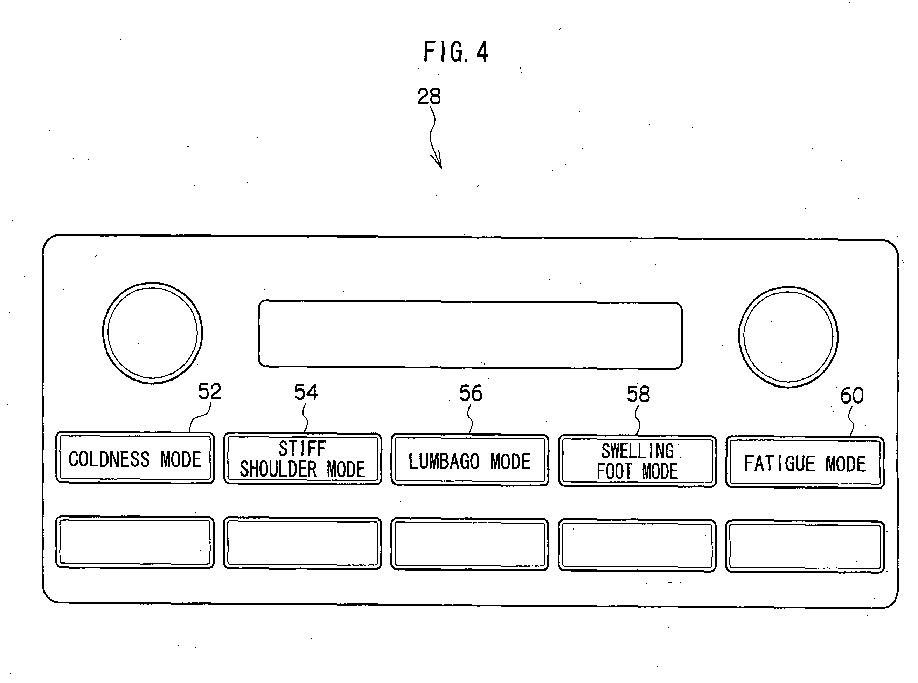

The operation panel 28 is arranged in an instrument panel 50 provided in the vehicle, for example, a center portion thereof, and is configured such as to have respective mode switches (a coldness mode switch 52, a stiff shoulder mode switch 54, a lumbago mode switch 56, a swelling foot mode switch 58 and a fatigue mode switch 60), as shown in Fig. 4. The operation panel 28 is configured such as to output a corresponding operation signal to the control circuit 30 mentioned below in the case that each of the mode switches is pushed by the crew.

The control circuit 30 is constituted by an electric circuit provided with CPU, ROM, RAM and the like, and is arranged at an appropriate position of the vehicle, for example, within the instrument panel 50 of the vehicle. The control circuit 50 is configured such as to discriminate a symptom (for example, excessive sensitivity to cold, stiff shoulder, lumbago, swelling foot, fatigue and the like) of the crew on the basis of the data or the signal output from the various sensors mentioned above and the operation signal output from the operation panel 28 mentioned above and selectively actuate a plurality of seat heaters 36, 38, 40 and 42 so as to generate heat. In this case, a detailed description of the motion of the control circuit 30 will be given below.

Next, a description will be given of an operation and an effect of the vehicle-use crew heating device 10 in accordance with the first exemplary embodiment of the invention as well as a motion thereof.

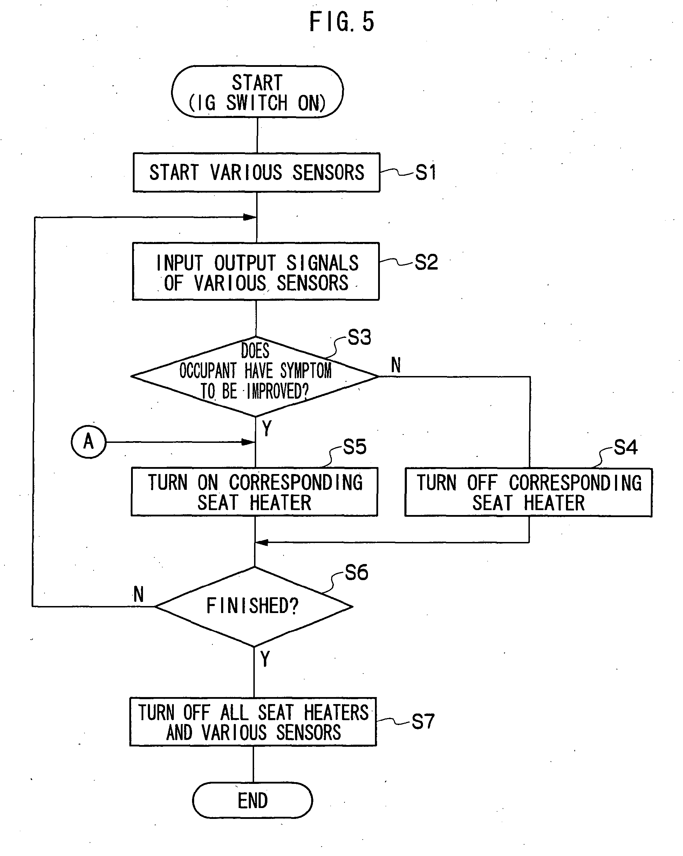

In the vehicle-use crew heating device 10 in accordance with the first exemplary embodiment of the invention, if an ignition switch of the vehicle is turned on, the control circuit 30 starts a process of a program shown by a flow chart in Fig. 5. The control circuit 30 first activates the various sensors (the camera 14, the infrared ray sensor 16, the heart rate sensor 18, the blood circulation sensor 20, the pressure sensitive sensors 22 and 24, and the pedal operation sensor 26) mentioned above in the case of starting the process of the program shown by the flow chart in Fig. 5 (a step S1).

The various sensors (the camera 14, the infrared ray sensor 16, the heart rate sensor 18, the blood circulation sensor 20, the pressure sensitive sensors 22 and 24, and the pedal operation sensor 26) activated by the control circuit 30 as mentioned above detect various states of the crew on the basis of their operations, and output the detected data or the detected signals to the control circuit 30.

That is, the camera 14 photographs the posture of the whole body, the motion state of the hand and the foot, the facial expression and the like of the crew seating on the vehicle-use seat 12, and outputs the photographed data to the control circuit 30, and the infrared ray sensor 16 detects the body temperature distribution of the crew seating on the vehicle-use seat 12 and outputs the data corresponding to the detected body temperature distribution to the control circuit 30. Further, the heart rate sensor 18 detects the heart rate of the crew seating on the vehicle-use seat 12 and outputs the heart rate data to the control circuit 30.

Further, the blood circulation sensor 20 measures the blood circulation of the shoulder portion of the seated crew and outputs the measured data to the control circuit 30. Further, the pressure sensitive sensor 22 measures the hardness of the muscle in the shoulder portion of the seated crew, and outputs the measured data to the control circuit 30, and the pressure sensitive sensor 24 measures the force (the seat pressure) pressed by the hip portion of the seated crew, and outputs the measured data to the control circuit 30. Further, the pedal operation sensor 26 detects the operation state of the operation pedal 48 (the operation state of any one or all of the accelerator pedal, the brake pedal and the clutch pedal) provided in the driver seat of the vehicle, and outputs the pedal operation signal to the control circuit 30 every time when the operation pedal 48 is operated.

The control circuit 30 inputs the detected data or the detected signals output from the various sensors (the camera 14, the infrared ray sensor 16, the heart rate sensor 18, the blood circulation sensor 20, the pressure sensitive sensors 22 and 24, and the pedal operation sensor 26) in the manner mentioned above (a step S2). Further, the control circuit 30 determines whether or not the crew has the symptom (for example, excessive sensitivity to cold, stiff shoulder, lumbago, swelling foot, fatigue and the like) to be improved on the basis of the detected data or the detected signal (a step S3).

At this time, for example, in the case that it is determined in the process of the step S3 that the crew does not have any symptom to be improved because the crew just gets on the vehicle (NO in the step S3), the control apparatus 30 maintains a state in which all the seat heaters 36, 38, 40 and 42 are turned off (a step S4), and gives way to a process of a step S6 mentioned below.

On the contrary, in the case that it is determined that the crew has the symptom to be improved (YES in the step S3), the control circuit 30 selectively actuates the seat heater arranged at the predetermined position according to the symptom in a plurality of seat heaters so as to generate heat (a step S5).

For example, in the case that it is determined on the basis of the data corresponding to the body temperature distribution output from the infrared ray sensor 16 that the temperature of the foot point of the seated crew is low, the control circuit 30 determines that the seated crew has the symptom of an excessive sensitivity to cold. Further, the control circuit 30 selectively actuates the seat heaters 40 and 42 arranged at the predetermined positions according to an excessive sensitivity to cold in a plurality of seat heaters so as to generate heat.

Accordingly, since the lumbar portion, which is thought to play the role of sensing the temperature of the whole of the human body is warmed up by the seat heater 40, thereby improving the function of the autonomic nerves, blood circulation to the whole body, particularly to deletions portions such as the hands and feet and the like is increased. Further, the thigh portion, which forms a pathway for blood circulation to the lower limbs, is warmed up by the seat heater 42, and the feet are warmed to the toes. Thereby, an excessive sensitivity to cold of the seated crew is improved.

Further, for example, in the case that it is determined on the basis of the measured data of the hardness of the muscle in the shoulder portion of the seated crew output from the pressure sensitive sensor 22, and the measured data of the blood circulation in the shoulder portion of the seated crew output from the blood circulation sensor 20 that the muscle in the shoulder portion of the seated crew is hard, and the blood circulation in the shoulder portion of the seated crew is small, the control circuit 30 determines that the seated crew has the symptom of stiff shoulder. Further, the control circuit 30 selectively actuates the seat heaters 36, 38 and 40 arranged at the predetermined positions according to stiff shoulder in a plurality of seat heaters so as to generate heat.

Accordingly, since the lumbar portion, which is thought to play the role of sensing the temperature of the whole of the human body, is warmed up by the seat heaters 38 and 40, thereby improving the function of the autonomic nerves, blood circulation to the whole body is increased. Further, since the shoulder portion of the seated crew that generates stiff shoulder is warmed up by the seat heater 36, and blood circulation is increased, it is possible to increase the discharge of metabolic decomposition products and increase the supply of oxygen and nutrients.Further, it is possible to expect an effect that a knotted muscle is loosened up by the heat. Thereby, stiff shoulder of the seated crew is improved.

Further, for example, the control circuit 30 calculates a body weight distribution of the seated crew on the basis of the measured data output according to the seating pressure of the hip portion of the seated crew from the pressure sensitive sensor 24 and estimates the seated posture of the seated crew from the body weight distribution. In the case that the control circuit 30 determines that the seating way imposes a burden on the lumbar, and a seating time (for example, converted by a time from a time when the measured data indicating the generation of the seating pressure is input from the pressure sensitive sensor 24) is long, the control circuit 30 determines that the seated crew has the symptom of lumbago.

In this case, the control circuit 30 may detect a reseating number of the seated crew on the basis of the measured data output from the pressure sensitive sensor 24, and assume that the seated crew has a sense of discomfort in the lumbar so as to determine that the seated crew has the symptom of lumbago, in the case that the reseating frequency is large.

Further, the control circuit 30 selectively actuates the seat heaters 38 and 40 arranged at the predetermined positions according to lumbago in a plurality of seat heaters so as to generate heat.

Accordingly, since the lumbar portion, which is thought to play the role of sensing the temperature of the whole of the human body, is warmed up by the seat heaters 38 and 40, thereby improving the function of the autonomic nerves, blood circulation to the whole body is increased. Further, since the lumbar portion of the seated crew that generates lumbago is warmed up by the seat heaters 38 and 40, and blood circulation is increased, it is possible to increase the discharge of metabolic decomposition products and increase the supply of oxygen and nutrients. Further, it is possible to expect the effect that knotted muscles are loosened up by the heat. Thereby, the lumbago of the seated crew is improved.

Further, for example, in the case that the control circuit 30 detects an operating frequency of the operation pedal 48 executed by the seated crew on the basis of a detecting frequency of the pedal operation signal output from the pedal operation sensor 26, and determines that the operating frequency is small (a kinetic momentum of the foot is small),the control circuit 30 determines that the seated crew has the symptom of swelling foot. Further, the control circuit 30 selectively actuates the seat heaters 40 and 42 arranged at the predetermined positions according to swelling foot in a plurality of seat heaters so as to generate heat.

Accordingly, since the lumbar portion, which is thought to play the role of sensing the temperature of the whole of the human body, is warmed up by the seat heater 40,thereby improving the function of the autonomic nerves, blood circulation to the whole body, particularly the deletions portions such as the hands and feet, is increased. Further, the thigh portion, which forms a pathway for blood circulation to the lower limbs, is warmed up by the seat heater 42, and the feet are warmed to the toes. Thereby, contracted muscles are loosened up; the functions of the lymph vessels are activated, and the swelling foot of the seated crew is improved.

Further, for example, the control circuit 30 detects a fluctuation of a breath and the heart rate on the basis of the measured data output from the heart rate sensor 18 and the data corresponding to the body temperature distribution output from the infrared ray sensor 16, and determines that the seated crew has the symptom of the fatigue in the case that the fluctuation of the breath and the heart rate forms a peculiar aspect to the fatigue.

In this case, the control circuit 30 may detect a skin temperature of a face of the seated crew on the basis of the body temperature distribution output from the infrared ray sensor 16, and combine the measured data output from the heart rate sensor 18 therewith, thereby measuring a duration of a stress of the seated crew. In the case that the duration of the stress of the seated crew is long, the control circuit 30 may determine that the seated crew has the symptom of the fatigue. Further, the control circuit 30 may determine a facial expression of the seated crew on the basis of the photographed data from the camera 14 and determine that the seated crew has the symptom of the fatigue in the case that it is determined that the seated crew has a fatigue facial expression.

Further, the control circuit 30 selectively actuates the seat heaters 36, 38, 40 and 42 arranged at the predetermined positions according to the fatigue so as to generate heat.

Accordingly, the shoulder portion, the lumbar portion and the thigh portion are respectively warmed up by the seat heaters 36, 38, 40 and 42. Therefore, it is possible to promote the blood circulation of approximately the whole body from the shoulder portion to the lumbar portion and the thigh portion, and to the lower limbs by extension, and the fatigue of the seated crew is improved. In this case, the control circuit 30 may specify the fatigue position from the posture of the whole body and the operating amount of the hand and the foot of the seated crew on the basis of the photographed data from the camera 14, and actuates only the seat heater positioned at the fatigue position so as to generate heat. ,

Subsequently, the control circuit 30 determines whether or not it finishes a series of program process, for example, by determining whether or not an ignition switch is turned off (a step S6), and is returned to the process of the step S2 mentioned above, in the case that the control circuit 30 determines that it does not finish the series of program process (NO in the step S6). Further, the process from the step S2 to the step S6 is repeatedly executed until the control circuit 30 determines that it finishes the series of program process.

At this time, in the case that the control circuit determines that the crew has the symptom to be improved in the process of the step S3 mentioned above, however, the symptom of the crew is improved by repeatedly executing the series of program process (a part of the body of the seated crew being warmed up by the process in the step S5) (NO in the step S3), the control circuit 30 turns off the corresponding seat heater in a plurality of seat heaters 36, 38, 40 and 42 (a step S4).

Further, in the first exemplary embodiment in accordance with the invention, in the case that each of the mode switches (the coldness mode switch 52, the stiff shoulder mode switch 54, the lumbago mode switch 56, the swelling foot mode switch 58 and the fatigue mode switch 60) of the operation panel 28 is selected so as to be pushed, during the time when the control circuit 30 executes the series of program process mentioned above, the control circuit 30 executes an interrupt processing of this operation so as to actuate the corresponding seat heater in a plurality of seat heaters 36, 38, 40 and 42 (an interrupt processing A).

That is, in the case that the coldness mode switch 52 is pushed, the control circuit 30 selectively actuates the seat heaters 40 and 42 arranged at the predetermined positions according to an excessive sensitivity to cold, in a plurality of seat heaters so as to generate heat. Further, in the case that the stiff shoulder mode switch 54 is pushed, the control circuit 30 selectively actuates the seat heaters 36, 38 and 40 arranged at the predetermined positions according to stiff shoulder in a plurality of seat heaters so as to generate heat.

Further, in the case that the lumbago mode switch 56 is pushed, the control circuit 30 selectively actuates the seat heaters 36, 38 and 40 arranged at the predetermined positions according to lumbago, in a plurality of seat heaters so as to generate heat. Further, in the case that the swelling foot mode switch 58 is pushed, the control circuit 30 selectively actuates the seat heaters 40 and 42 arranged at the predetermined positions according to swelling foot in a plurality of seat heaters so as to generate heat.

Further, in the case that the fatigue mode switch 60 is pushed, the control circuit 30 selectively actuates the seat heaters 36, 38, 40 and 42 arranged at the predetermined positions according to the fatigue so as to generate heat. In this case, the operation in the case of selectively actuating the seat heaters 36, 38, 40 and 42 so as to generate heat is the same as mentioned above, and a description thereof will be omitted. Further, in this case, the structure may be made such that an operation intent of the crew is detected in accordance with a voice recognition and each of the seat heaters is actuated.

Further, in the case that the control circuit 30 determines that it finishes all the program processes (YES in the step S6), the control circuit 30 turns off all the seat heaters 36, 38, 40 and 42 and the various sensors (the camera 14, the infrared ray sensor 16, the heart rate sensor 18, the blood circulation sensor 20, the pressure sensitive sensors 22 and 24, and the pedal operation sensor 26) (a step S7), and finishes the series of program process.

As in detail mentioned above, in the vehicle-use crew heating device 10 in accordance with the first exemplary embodiment of the invention, a plurality of seat heaters 36, 38, 40 and 42 are divisionally arranged in the vehicle-use seat 12 according to a plurality of symptoms (for example, excessive sensitivity to cold, stiff shoulder, lumbago, swelling foot, fatigue and the like) of the seated crew that are to be improved, and the seat heaters 36, 38, 40 and 42 are selectively operated according to the symptom (for example, excessive sensitivity to cold, stiff shoulder, lumbago, swelling foot, fatigue and the like) of the seated crew that are to be improved, and a local region corresponding to the symptom to be improved of the seated crew is warmed up (receives thermal stimulation). Accordingly, it is possible to improve the various symptoms (for example, excessive sensitivity to cold, stiff shoulder, lumbago, swelling foot, fatigue and the like) which are different according to the situation of the crew, and it is possible to provide a more comfortable vehicle interior space.

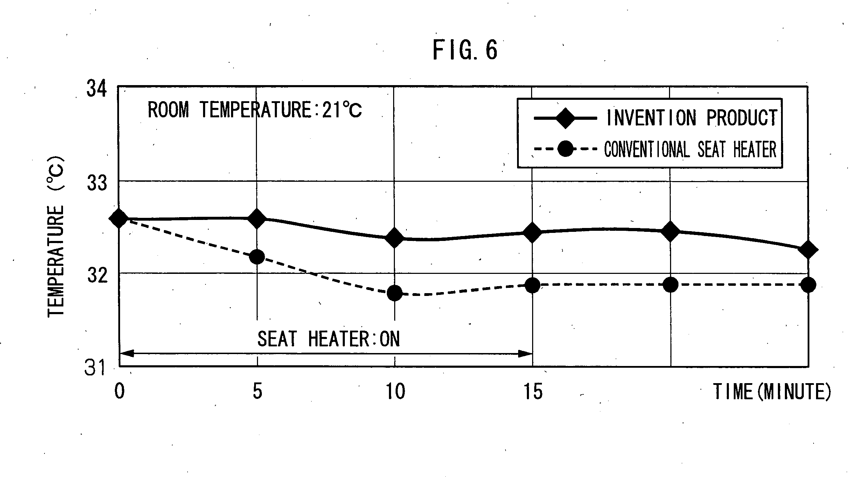

In this case, Fig. 6 shows a comparison data between a case that the crew is warmed up in accordance with the excessive sensitivity to cold mode by the vehicle-use crew heating device 10 in accordance with the first exemplary embodiment of the invention, and a case that the crew is warmed up by the conventional seat heater apparatus for the vehicle. In this case, the conventional seat heater apparatus for the vehicle is configured such as to heat an approximately all the area of the seat back and approximately all the area of the seat cushion of the vehicle-use seat by the seat heater. Further, in this comparison, a test subject becoming aware of an excessive sensitivity to cold at a room temperature of 21°C is seated on each of the vehicle-use seats, each of the seat heaters is turned on only for fifteen minutes from the start, and the temperature at the foot point of the test subject is measured by a thermal photography.

As shown in Fig. 6, in the case of warming up the crew in accordance with an excessive sensitivity to cold mode by the vehicle-use crew heating device 10 in accordance with the first exemplary embodiment of the invention, the temperature of the foot point becomes about 0.8°C higher in comparison with the case of warming up the crew by the conventional seat heater apparatus for the vehicle. Further, after turning off the seat heater, the same result can be also obtained. As mentioned above, in accordance with the vehicle-use crew heating device 10 on the basis of the first exemplary embodiment of the invention, it is possible to obtain a high symptom improving effect in comparison with the case of warming up the whole body as in the comparative example, by locally heating the region corresponding to the symptom to be improved of the seated crew.

Further, in the vehicle-use crew heating device 10 in accordance with the first exemplary embodiment of the invention, the state of the seated crew is detected by the various sensors (the camera 14, the infrared ray sensor 16, the heart rate sensor 18, the blood circulation sensor 20, the pressure sensitive sensors 22 and 24, and the pedal operation sensor 26). Accordingly, it is possible to detect the various symptoms (for example, excessive sensitivity to cold, stiff shoulder, lumbago, swelling foot, fatigue and the like) as the symptom to be improved of the seated crew according to the situation of the crew.

Further, in the vehicle-use crew heating device 10 in accordance with the first exemplary embodiment of the invention, the control circuit 30 selectively actuates a plurality of seat heaters 36, 38, 40 and 42 on the basis of the result of detection of the various sensors mentioned above. Accordingly, a suitable region according to the symptom to be improved of the seated crew is locally warmed up (receives thermal stimulation). In accordance with the structure mentioned above, it is possible to improve the various symptoms (for example, excessive sensitivity to cold, stiff shoulder, lumbago, swelling foot, fatigue and the like) which are different according to the situation of the crew, and it is possible to provide a further more comfortable vehicle interior space.

Further, the switch operation or the like of the seated crew becomes unnecessary by automatically actuating a plurality of seat heaters 36, 38, 40 and 42 on the basis of the result of detection of the various sensors mentioned above, and a good usability is obtained.

On the other hand, in the vehicle-use crew heating device 10 in accordance with the first exemplary embodiment of the invention, if the operation signal is output from the operation panel according to the operation intent of the seated crew, the control circuit 30 selectively actuates a plurality of seat heaters 36, 38, 40 and 42 on the basis of the operation signal output from the operation panel 28. Accordingly, the crew expresses the operation intent (by for example, operating a switch) for improving the symptom that the crew feels at that moment, whereby the region coinciding with this operation intent is locally warmed up (receives thermal stimulation). Thereby, it is possible for the crew to improve the symptom (for example, excessive sensitivity to cold, stiff shoulder, lumbago, swelling foot, fatigue and the like) which he or she feels at that moment, by his or her own will, and it is possible to provide a more comfortable vehicle interior space.

Further, in the vehicle-use crew heating device 10 in accordance with the first exemplary embodiment of the invention, each of a plurality of seat heaters 36, 38, 40 and 42 is configured such that the temperature of the center portion C becomes higher than the temperature of the peripheral portion thereof. Accordingly, it is possible to set the warming up region of the body of the seated crew to within a small area, and it is possible to more locally warm up the region corresponding to the symptom (for example, excessive sensitivity to cold, stiff shoulder, lumbago, swelling foot, fatigue and the like) to be improved of the seated crew. Therefore, it is possible to improve the various symptoms (for example, excessive sensitivity to cold, stiff shoulder, lumbago, swelling foot, fatigue and the like) which are different according to the situation of the crew, without imparting a stuffy feeling to the seated crew, and it is possible to provide a more comfortable vehicle interior space.

### [Second Exemplary Embodiment]

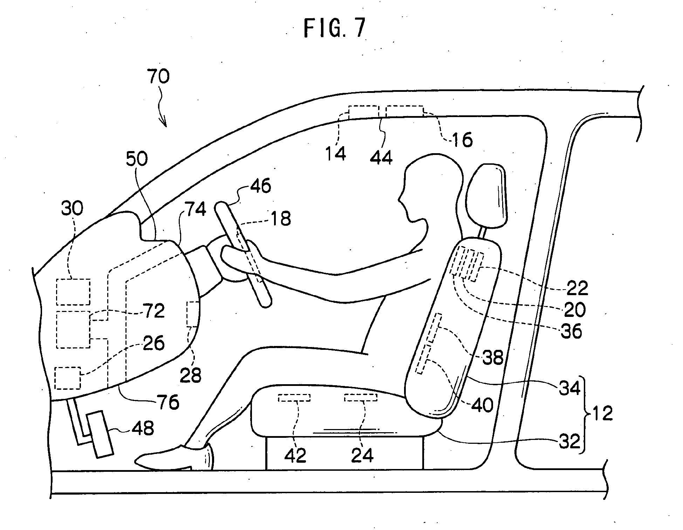

Next, a description will be given of a second exemplary embodiment in accordance with the invention with reference to Figs. 7 to 10.

A vehicle-use crew heating device 70 in accordance with the second exemplary embodiment of the invention shown in Fig. 7 is configured such that an air conditioning unit 72 serving as a crew-sensed-temperature adjusting means is additionally provided in the vehicle-use crew heating device 10 in accordance with the first exemplary embodiment mentioned above. Accordingly, in the vehicle-use crew heating device 70 in accordance with the second exemplary embodiment of the invention, the same reference numerals are used for the same structures as those of the vehicle-use crew heating device 10 in accordance with the first exemplary embodiment mentioned above, and a description thereof will be omitted.

In this case, the air conditioning unit 72 is configured such that a warm air or a cool air can be blown from a face blowout port 74 and a foot blowout port 76. Further, the control circuit 30 is configured such as to discriminate the symptom (for example, excessive sensitivity to cold, stiff shoulder, lumbago, swelling foot, fatigue and the like) of the crew on the basis of data or the signal output from the various sensors and the operation signal output from the operation panel 28 mentioned above, and actuate a plurality of seat heaters 36, 38, 40 and 42 and the air conditioning unit 72 in an interlocking manner.

Next, a description will be given of an operation and an effect of the vehicle-use crew heating device 70 in accordance with the second exemplary embodiment of the invention as well as its motion. In this case, in the following description, a description will be given only of a different motion from the first exemplary embodiment of the invention mentioned above:

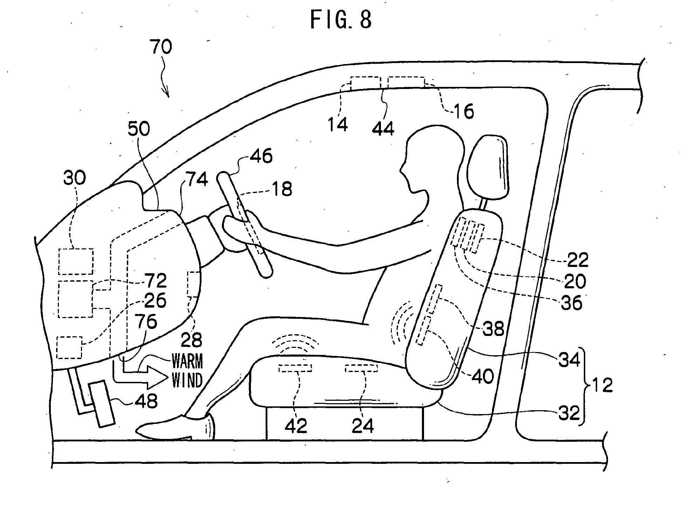

In the vehicle-use crew heating device 70 in accordance with the second exemplary embodiment of the invention shown in Fig. 8, in the case that it is determined that the seated crew has the symptom of an excessive sensitivity to cold, or in the case that the coldness mode switch 52 (refer to Fig. 4) is pushed, the control circuit 30 selectively actuates the seat heaters 40 and 42 arranged at the predetermined positions according to an excessive sensitivity to cold in a plurality of seat heaters so as to generate heat. Further, the control circuit 30 accessorily actuates the air conditioning unit 72 working therewith, and blows out a warm wind from the foot blowout port 76.

In accordance with this structure, it is possible to locally warm up the appropriate region coinciding with the symptom to be improved of the seated crew by the seat heaters 40 and 42 while it is possible to accessorily warm up the foot point portion which can not be directly warmed up by the seat heaters 40 and 42 mentioned above in the body of the seated crew by the warm wind of the air conditioning unit 72. Accordingly, the vehicle interior space becomes further more comfortable by employing the structure mentioned above.

In this case, at this time, in the case that it is determined on the basis of the data according to the body temperature distribution output from the infrared ray sensor 16 that the temperature in the foot point of the seated crew is lower than a predetermined value (for example, in the case that it is determined that an excessive sensitivity to cold can not be improved only by the operation of the seat heaters 40 and 42), or in the case that it is determined that an excessive sensitivity to cold improving effect is small even after a predetermined time has elapsed, in spite that only the seat heaters 40 and 42 are actuated, the air conditioning unit 72 may be accessorily actuated and the warm wind may be blown out from the foot blowout port 76.

Further, in the vehicle-use crew heating device 70 in accordance with the second exemplary embodiment of the invention, in the case that it is determined that the seated crew has the symptom of an excessive sensitivity to cold, or in the case that the coldness mode switch 52 (refer to Fig. 4) is pushed, as mentioned above, the control circuit 30 selectively actuates the seat heaters 40 and 42 arranged at the predetermined position according to an excessive sensitivity to cold in a plurality of seat heaters so as to generate heat, however, in the case that it is determined that the crew is just after getting on the vehicle (for example, the crew is just after getting on the vehicle in the winter season in which the temperature within the passenger room is low), and the passenger room is thereafter set to the comfortable temperature, the control circuit 30 accessorily actuates the air conditioning unit 72 as follows.

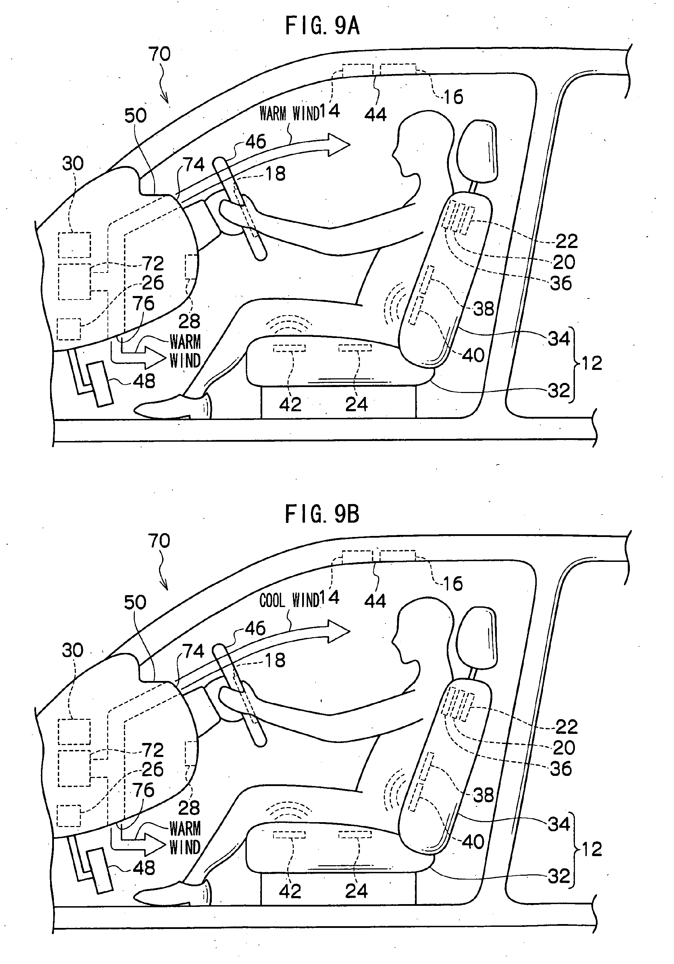

That is, the control circuit 30 assumes a case that a hot flash feeling or a sleepy feeling is generated at a time of selectively actuating the seat heaters 40 and 42 arranged at the predetermined positions according to an excessive sensitivity to cold in a plurality of seat heaters so as to generate heat, and accessorily actuates the air conditioning unit 72 just after getting on the vehicle (for example, just after getting on the vehicle in the winter season in which the temperature within the passenger room is low), as shown in Fig. 9A, thereby blowing out the warm wind from the face blowout port 74 and the foot blowout port 76. Thereafter, the control circuit 30 switches the air conditioning unit 72 as shown in Fig. 9B after the passenger room becomes a suitable temperature, thereby blowing out the cool wind from the face blowout port 74 while the control circuit 30 blows out the warm wind from the foot blowout port 76.

In accordance with the structure mentioned above, it is possible to locally warm up the appropriate region coinciding with the symptom to be improved of the seated crew by the seat heaters 40 and 42 while it is possible to suitably keep the sensible temperature of the crew. Accordingly, the vehicle interior space becomes further more comfortable by employing the structure mentioned above.

Further, in the vehicle-use crew heating device 70 in accordance with the second exemplary embodiment of the invention, in the case that the control circuit 30 determined that the seated crew has the symptom of lumbago, or in the case that the lumbago mode switch 56 (refer to Fig. 4) is pushed, as mentioned above, the control circuit 30 selectively actuates the seat heaters 36, 38 and 40 arranged at the predetermined positions according to lumbago in a plurality of seat heaters so as to generate heat, however, for example, in the case that the seat heaters 36, 38 and 40 are used in the summer season or the like, the control circuit 30 actuates the air conditioning unit 72 in an interlocking manner as follows at a time when the crew is just after getting on the vehicle (for example, in a state in which the temperature within the passenger room is high), and the passenger room is thereafter set to the comfortable temperature.

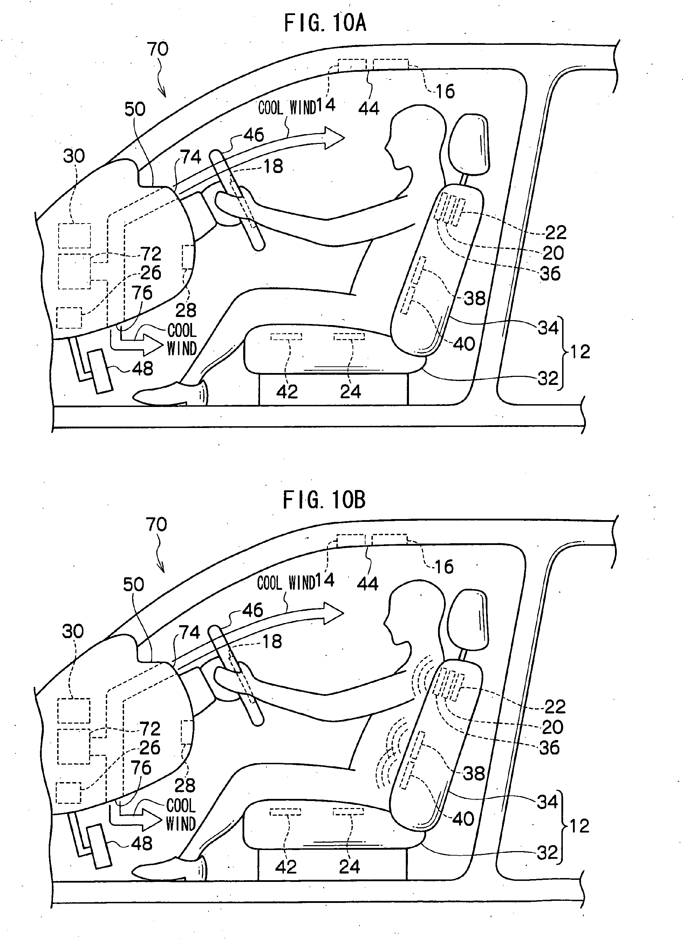

That is, even in the case that it is determined that the seated crew has the symptom of lumbago in the summer season or the like, or even in the case that the lumbago mode switch 56 (refer to Fig. 4) is pushed, the passenger room is cooled by actuating the air conditioning unit 72 so as to blow out the cool wind from the face blowout port 74 and the foot blowout port 76 without actuating the seat heaters 36, 38, 40 and 42, as shown in Fig. 10A, just after getting on the vehicle (for example, in the state in which the temperature within the passenger room is high). Further, after the passenger room becomes the suitable temperature, the control circuit 30 actuates the seat heaters 36, 38 and 40 for improving lumbago so as to generate heat, while the control circuit 30 maintains the operation of the air conditioning unit 72, as shown in Fig. 10B.

In accordance with the structure mentioned above, it is possible to locally warm up the appropriate region coinciding with the symptom to be improved of the seated crew by the seat heaters 36, 38 and 40 while it is possible to keep the temperature within the passenger room at the suitable temperature corresponding to the season by the air conditioning unit 72. Accordingly, the vehicle interior space becomes further more comfortable by employing the structure mentioned above.

### [Third Exemplary Embodiment]

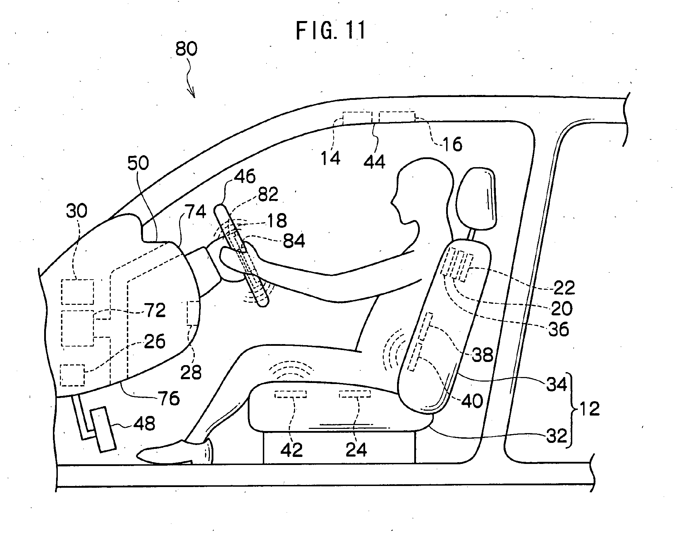

Next, a description will be given of a third exemplary embodiment in accordance with the invention with reference to Fig. 11.

A vehicle-use crew heating device 80 in accordance with the third exemplary embodiment of the invention shown in Fig. 11 is configured such that the vehicle-use crew heating device 70 in accordance with the second exemplary embodiment mentioned above is additionally provided with a steering wheel heater 82 serving as a crew-sensed-temperature adjusting means, and a blood circulation sensor 84 serving as a crew state detecting means. Accordingly, in the vehicle-use crew heating device 80 in accordance with the third exemplary embodiment of the invention, the same reference numerals are used for the same structures as those of the vehicle-use crew heating device 70 in accordance with the second exemplary embodiment mentioned above, and a description thereof will be omitted.

In this case, the steering wheel heater 82 is configured such as to be incorporated in the steering wheel 46 of the vehicle, and be actuated so as to generate heat, in the same manner as each of the seat heaters 36, 38, 40 and 42 mentioned above. Further, the blood circulation sensor 84 is integrally provided in the steering wheel 46, and is configured such as to measure the blood circulation of the hand point of the seated crew and output the measured date to the control circuit 30.

Further, the control circuit 30 is configured such as to discriminate the symptom (for example, excessive sensitivity to cold, stiff shoulder, lumbago, swelling foot, fatigue and the like) of the crew on the basis of the data or the signal output from the various sensors including the blood circulation sensor 84 and the operation signal output from the operation panel 28 mentioned above, and actuate a plurality of seat heaters 36, 38, 40 and 42, the steering wheel heater 82 and the air conditioning unit 72 in an interlocking manner.

Next, a description will be given of an operation and an effect of the vehicle-use crew heating device 80 in accordance with the third exemplary embodiment of the invention as well as a motion thereof. In this case, in the following description, a description will be given below only of a different motion from the motion of the first exemplary embodiment and the second exemplary embodiment in accordance with the invention mentioned above.

In the vehicle-use crew heating device 80 in accordance with the third exemplary embodiment of the invention shown in Fig. 11, in the case that it is determined that the seated crew has the symptom of an excessive sensitivity to cold, or in the case that the coldness mode switch 52 (refer to Fig. 4) is pushed, the control circuit 30 selectively actuates the seat heaters 40 and 42 arranged at the predetermined positions according to an excessive sensitivity to cold in a plurality of seat heaters so as to generate heat. Accordingly, since the lumbar portion assumed to play a role of feeling the temperature of the whole of the human body is warmed up by the seat heater 40, and the function of the autonomic nerve is adjusted, the blood circulation to the whole body, particularly to the deletions portion such as the hand, the foot and the like is increased. Further, the thigh portion forming the pass way of the blood circulation to the lower limb is warmed up by the seat heater 42, and the foot point is warmed up.

Further, in the case that the control circuit 30 determines on the basis of the measured date from the blood circulation sensor 84 incorporated in the steering wheel 46 that the temperature of the hand point of the seated crew is low, the control circuit 30 actuates the steering wheel 82 in an interlocking manner so as to generate heat. Accordingly, the hand point portion assumed as the position at which the blood circulation is small in the crew having an excessive sensitivity to cold is warmed up by the steering wheel 82. Therefore, it is possible to promote the blood circulation from the lumbar portion to the thigh portion, and the lower limb end portion by extension, and the blood circulation of the hand point of the seated crew is also promoted, so that an excessive sensitivity to cold of the seated crew is improved.

As mentioned above, in accordance with the vehicle-use crew heating device 80 on the basis of the third exemplary embodiment of the invention, it is possible to accessorily warm up the hand point portion which can not be directly warmed up by the seat heaters 36, 38, 40 and 42 mentioned above in the body of the seated crew by the steering wheel heater 82. Accordingly, an excessive sensitivity to cold of the seated crew is improved by employing the structure mentioned above, and the vehicle interior space becomes further more comfortable.

In this case, in the structure mentioned above, the temperature of the hand point of the seated crew is determined on the basis of the measured date from the blood circulation sensor 84 incorporated in the steering wheel 46, however, the structure may be made such that the temperature of the hand point of the seated crew is detected and determined by the infrared ray sensor 16 mentioned above. Further, the structure may be made such that the warm wind is blown to the hand point of the crew from the face blowout port 74 by accessorily actuating the air conditioning unit 72 mentioned above in place of the steering wheel heater 82.

### [Fourth Exemplary Embodiment]

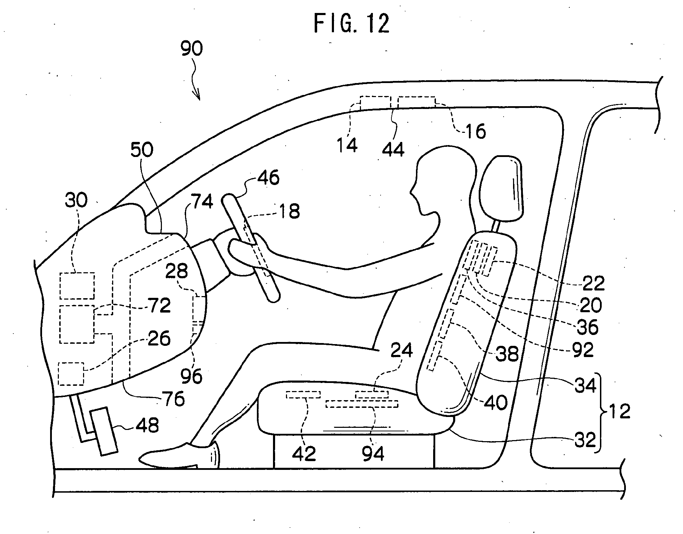

Next, a description will be given of a fourth exemplary embodiment in accordance with the invention with reference to Figs. 12 to 14.

A vehicle-use crew heating device 90 in accordance with the fourth exemplary embodiment of the invention shown in Fig. 12 is configured such that the vehicle-use crew heating device 70 in accordance with the second exemplary embodiment mentioned above is additionally provided with accessorily heating heaters 92 and 94 serving as a crew-sensed-temperature adjusting means, and an interior air sensor 96. Accordingly, in the vehicle-use crew heating device 90 in accordance with the fourth exemplary embodiment of the invention, the same reference numerals are used for the same structures as those of the vehicle-use crew heating device 70 in accordance with the second exemplary embodiment mentioned above, and a description thereof will be omitted.

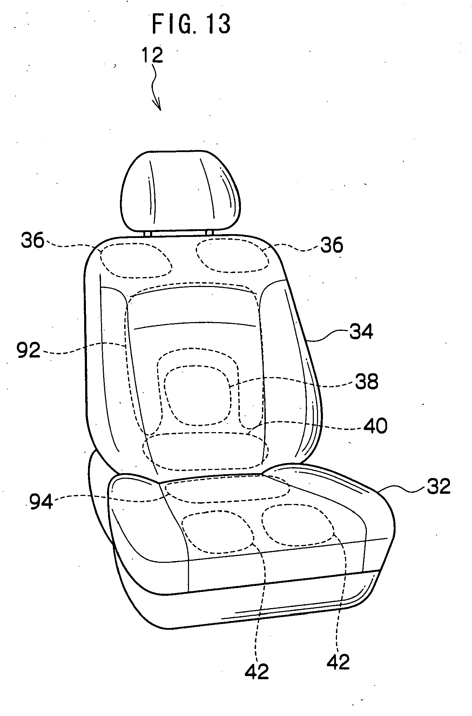

In this case, the accessorily heating heater 92 is provided in the other area (mainly near a back of the crew and a region supporting both sides of the lumbar portion) than the area in which the seat heaters 36, 38 and 40 are provided, in the seat back 34 of the vehicle-use seat 12, as shown in Fig. 13, and the accessorily heating heater 94 is provided in the other area (a region supporting the hip portion of the crew) than the area in which the seat heater 42 is provided, in the seat cushion 32 of the vehicle-use seat 12. Further, the accessorily heating heaters 92 and 94 are structured such as to be actuated in the same manner as each of the seat heaters 36, 38, 40 and 42 mentioned above so as to generate heat. Further, the interior air sensor 96 shown in Fig. 12 is arranged, for example, integrally in the instrument panel 50, and is configured such as to output the signal corresponding to the temperature within the passenger room to the control circuit 30.

Further, the control circuit 30 is configured such as to discriminate the symptom (for example, excessive sensitivity to cold, stiff shoulder, lumbago, swelling foot, fatigue and the like) of the crew on the basis of the data or the signal output from the various sensors mentioned above, and the operation signal output from the operation panel 28 mentioned above so as to actuate a plurality of seat heaters 36, 38, 40 and 42, and actuate the accessorily heating heaters 92 and 94 in an interlocking manner on the basis of the signal output from the interior air sensor 96.

Next, a description will be given of an operation and an effect of the vehicle-use crew heating device 90 in accordance with the fourth exemplary embodiment of the invention as well as a motion thereof.

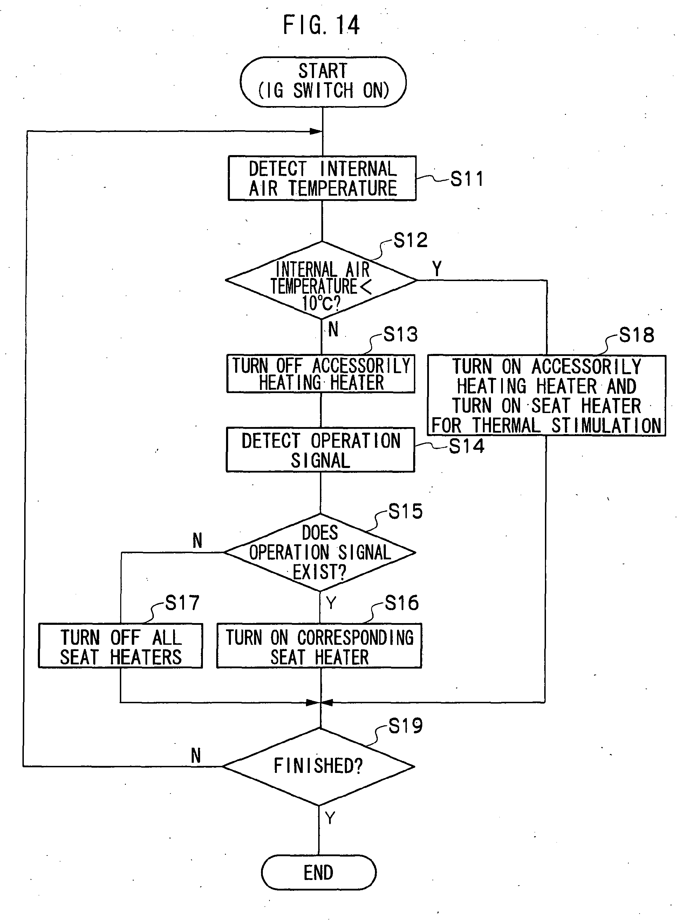

In the vehicle-use crew heating device 90 in accordance with the fourth exemplary embodiment of the invention, if the ignition switch of the vehicle is turned on, the control circuit 30 starts a process of a program shown by a flow chart in Fig. 14. If the control circuit 30 starts the process of the program shown by the flow chart in Fig. 14, the control circuit 30 detects the internal air temperature on the basis of the signal output from the internal air sensor 96 (a step S 11), and determines whether or not the temperature (the internal air temperature) within the vehicle interior space is lower than 10°C (a step S12).

Further, in the case that the control circuit 30 determines that the temperature (the internal air temperature) within the vehicle interior space is lower than 10°C (YES in the step S12), the control circuit 30 actuates both of the accessorily heating heaters 92 and 94, and the seat heaters 36, 38, 40 and 42 for the thermal stimulation so as to generate heat (a step S 18). Accordingly, even in the case that the temperature within the vehicle interior space is low, it is possible to improve the heating performance.

Subsequently, the control circuit 30 determines whether or not a series of program process is finished (a step S 19), for example, by determining whether or not the ignition switch is turned off, and in the case that the control circuit 30 determines that the series of program process is not finished (NO in the step S 19), the control circuit 30 is returned, to the process in the step S11 mentioned above.

Further, if the temperature within the vehicle interior space is increased, for example, by actuating the air conditioning unit 72 of the vehicle or the like during the repeated process execution of the step S11, the step S12, the step S 18 and the step S 19, as mentioned above, the control circuit 30 determines that the temperature (the internal air temperature) within the vehicle interior space is 10°C or more (NO in the step S12).

In this case, the control circuit 30 turns off the accessorily heating heaters 92 and 94 (a step S 13), and detects the operation signal output from the operation panel 28 (a step S14). Subsequently, the control circuit 30 determines whether or not the operation signal output from the operation panel 28 is provided (a step S 15), and sets the not-corresponding seat heater in a plurality of seat heaters 36, 38, 40 and 42 to a switch-off state and sets only the corresponding seat heater to a heat generating state (a step S16), in the case that the operation signal output from the operation panel 28 exists (YES in the step S15).

Accordingly, the appropriate region coinciding with the symptom to be improved of the seated crew is warmed up (is exposed to the thermal stimulation) by a plurality of seat heaters 36, 38, 40 and 42. On the other hand, in the case that the operation signal output from the operation panel 28 does not exist (NO in the step S15), all the seat heaters 36, 38, 40 and 42 are set to the switch-off state (a step S17).

Further, the control circuit 30 determines whether or not the series of program process is finished (a step S 19), for example, by determining whether or not the ignition switch is turned off, in the same manner as mentioned above, and is returned to the process of the step S11 mentioned above in the case that the control circuit 30 determines that the series of program process is not finished (NO in the step S 19). Further, the process in the steps S11 to S 19 mentioned above is repeatedly executed until the control circuit 30 determines that the series of program process is finished

As mentioned above, in accordance with the vehicle-use crew heating device 90 on the basis of the fourth exemplary embodiment of the invention, it is possible to achieve both of a heating performance at an extreme cold time, and an effect of a thermal stimulation by setting the accessorily heating heaters 92 and 94 covering the seat heaters 36, 38, 40 and 42 for the thermal stimulation and the other portions, and properly using them according to the temperature within the vehicle interior space as mentioned above. The vehicle interior space becomes further more comfortable by employing the structure mentioned above.

### [Fifth Exemplary Embodiment]

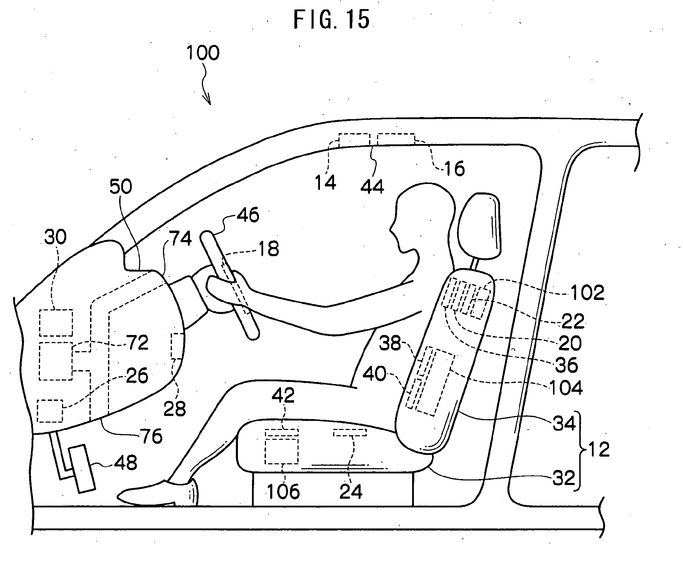

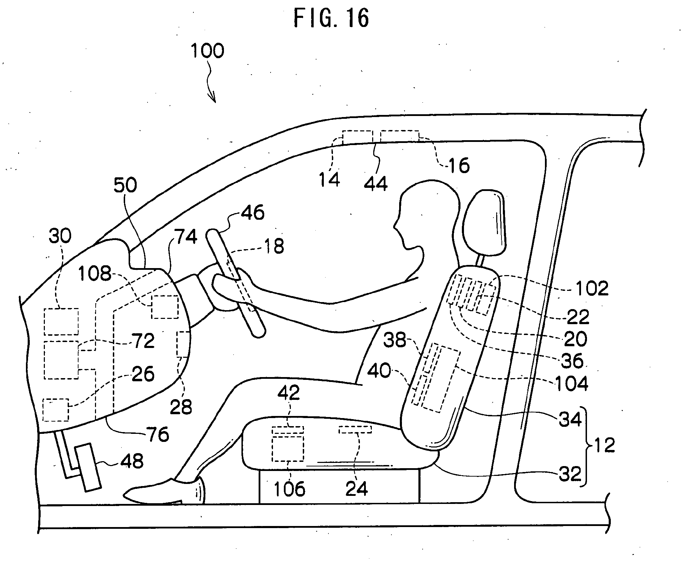

Next, a description will be given of a fifth exemplary embodiment in accordance with the invention with reference to Figs. 15 and 16.

A vehicle-use crew heating device 100 in accordance with the fifth exemplary embodiment of the invention shown in Fig. 15 is configured such that the vehicle-use crew heating device 70 in accordance with the second exemplary embodiment mentioned above is additionally provided with massaging devices 102, 104 and 106 serving as a relaxation supplying means. Accordingly, in the vehicle-use crew heating device 100 in accordance with the fifth exemplary embodiment of the invention, the same reference numerals are used for the same structures as those of the vehicle-use crew heating device 70 in accordance with the second exemplary embodiment mentioned above, and a description thereof will be omitted.

In this case, the massaging device 102 is provided at a region supporting the shoulder portion of the seated crew in the seat back 34 of the vehicle-use seat 12 (a position with which the shoulder portion of the seated crew is brought into contact), the massaging device 104 is provided at a region supporting the lumbar portion of the seated crew in the seat back 34 of the vehicle-use seat 12 (a position with which the lumbar portion of the seated crew is brought into contact), and the massaging device 106 is provided at a region supporting the thigh portion of the seated crew in the seat cushion 32 (a position with which the thigh portion of the seated crew is brought into contact). Each of the massaging devices 102, 104 and 106 is configured such as to carry out a massaging motion and a beating motion.

Further, the control circuit 30 is configured such as to discriminate the symptom (for example, excessive sensitivity to cold, stiff shoulder, lumbago, swelling foot, fatigue and the like) of the crew on the basis of the data or the signal output from the various sensors mentioned above, and the operation signal output from the operation panel 28 mentioned above so as to actuate a plurality of seat heaters 36, 38, 40 and 42, the massaging devices 102, 104 and 106 and the air conditioning unit 72 in an interlocking manner.

Next, a description will be given of an operation and an effect of the vehicle-use crew heating device 100 in accordance with the fifth exemplary embodiment of the invention as well as a motion thereof. In this case, in the following description, a description will be given only of the different motion from the first exemplary embodiment and the second exemplary embodiment in accordance with the invention mentioned above.

In the vehicle-use crew heating device 100 in accordance with the fifth exemplary embodiment of the invention, in the case that the control circuit 30 determines that the seated crew has the symptom of an excessive sensitivity to cold, or in the case that the coldness mode switch 52 (refer to Fig. 4) is pushed, the control circuit 30 selectively actuates the seat heaters 40 and 42 arranged at the predetermined positions according to an excessive sensitivity to cold in a plurality of seat heaters so as to generate heat. Accordingly, the lumbar portion assumed to be the position in which a lot of blood vessels gather together in the human body is warmed up by the seat heater 40, and the thigh portion in the lower limb assumed to be the position in which the blood circulation is small in the crew having an excessive sensitivity to cold is warmed up by the seat heater 42.

Further, in the case that the control circuit 30 determines on the basis of the data according to the blood circulation in the foot point of the seated crew output from the infrared ray sensor 16 that the temperature of the foot point of the seated crew is low, the control circuit 30 actuates the massaging devices 104 and 106 in an interlocking manner. Accordingly, the lumbar portion is massaged by the massaging device 104, and the thigh portion is massaged by the massaging device 106. Therefore, the blood circulation from the lumbar portion to the thigh portion, and to the lower limb end portion by extension is more promoted, an excessive sensitivity to cold of the seated crew is rapidly improved, and the relaxation generated by the massaging is supplied to the seated crew.

Further, in the case that the control circuit 30 determines that the seated crew has the symptom of stiff shoulder, or the stiff shoulder mode switch 54 (refer to Fig. 4) is pushed, the control circuit 30 selectively actuates the seat heaters 36, 38 and 40 arranged at the predetermined positions corresponding to stiff shoulder in a plurality of seat heaters so as to generate heat. Accordingly, since the lumbar portion assumed to play a role of sensing the temperature of the whole of the human body is warmed up by the seat heaters 38 and 40, and the function of the autonomic nerve is adjusted, the blood circulation of the whole body is increased. Further, since the shoulder portion of the seated crew corresponding to the generating position of stiff shoulder is warmed up by the seat heater 36, and the blood circulation is increased, the discharge of the metabolic decomposition product and the supply of the oxygen and the foodstuff are increased. Further, it is possible to expect the effect that the hard-set muscle is loosened up by the heat. Therefore, stiff shoulder of the seated crew is improved.

Further, in the case that the control circuit 30 determines on the basis of the measured date of the hardness of the muscle in the shoulder portion of the seated crew output from the pressure sensitive sensor 22, and the measured date of the blood circulation in the shoulder portion of the seated crew output from the blood sensor 20 that the muscle in the shoulder portion of the seated crew is considerably hard and the blood circulation of the shoulder portion of the seated crew is considerably small, the control circuit 30 determines that stiff shoulder of the seated crew is heavy and actuates the massaging devices 102 and 104 in an interlocking manner. Accordingly, the lumbar portion is massaged by the massaging device 104, and the shoulder portion is massaged by the massaging device 102. Accordingly, the relaxation by the massaging is supplied to the seated crew, as well as the blood circulation from the lumbar portion to the shoulder portion is more promoted, and stiff shoulder of the seated crew is rapidly improved.

Further, in the case that the control circuit 30 determines that the seated crew has the symptom of lumbago, or in the case that the lumbago mode switch 56 (refer to Fig. 4) is pushed, the control circuit 30 selectively actuates the seat heaters 38 and 40 arranged at the predetermined positions according to lumbago in a plurality of seat heaters so as to generate heat. Accordingly, since the lumbar portion assumed to play a role of sensing the temperature of the whole of the human body is warmed up by the seat heaters 38 and 40, and the function of the autonomic nerve is adjusted, the blood circulation to the whole body is increased. Further, since the lumbar portion of the seated crew corresponding to the generating position of lumbago is warmed up by the seat heaters 38 and 40, and the blood circulation is increased, the discharge of the metabolic decomposition product and the supply of the oxygen and the foodstuff are increased. Further, it is possible to expect the effect that the hard-set muscle is loosened up by the heat. Therefore, lumbago of the seated crew is improved.

Further, in the case that the control circuit 30 determines on the basis of the measured date output according to the seating pressure of the hip portion of the seated crew from the pressure sensitive sensor 24 that the seating way imposes a significant burden on the lumbar, and the seating time (for example, converted by the time from the time when the measured data indicating the generation of the seating pressure is input from the pressure sensitive sensor 24) is considerably long, the control circuit 30 determines that lumbago of the seated crew is severe, and actuates the massaging device 104. Accordingly, the lumbar portion 104 is massaged by the massaging device 104. Therefore, the relaxation generated by the massaging is supplied to the seated crew, as well as the blood circulation of the lumbar portion is more promoted, and lumbago of the seated crew is rapidly improved.

Further, in the case that the control circuit 30 determines that the seated crew has the symptom of swelling foot, or in the case that swelling foot mode switch (refer to Fig 4) is pushed, the control circuit 30 selectively actuates the seat heaters 40 and 42 arranged at the predetermined positions according to swelling foot in a plurality of seat heaters so as to generate heat. Accordingly, since the lumbar portion assumed to play a role of sensing the temperature of the whole of the human body is warmed up by the seat heater 40, and the function of the autonomic nerve is adjusted, the blood circulation to the whole body is increased, the blood circulation to the whole body, particularly to the deletions portion such as the hand, the foot and the like is increased. Further, the thigh portion forming the pass way of the blood circulation to the lower limb is warmed up by the seat heater 42 and the foot point is warmed up. Accordingly, the contracted muscle is loosened up, the function of the lymph vessel is activated, and swelling foot of the seated crew is improved.

Further, in the case that the control circuit 30 detects the operating frequency of the operation pedal 48 executed by the seated crew on the basis of the detecting frequency of the pedal operation signal output from the pedal operation sensor 26, and determines that the operating frequency is extremely small (the kinetic momentum of the foot is small), the control circuit 30 determines that swelling foot of the seated crew is severe, and actuates the massaging devices 104 and 106 in an interlocking manner. Accordingly, the lumbar portion is massaged by the massaging device 104, and the thigh portion is massaged by the massaging device 106. Therefore, the relaxation generated by the massaging is supplied to the seated crew, as well as the blood circulation from the lumbar portion to the thigh portion and to the lower limb end portion by extension is more promoted, and swelling foot of the seated crew is rapidly improved.

Further, in the case that the control circuit 30 determines that the seated crew has the symptom of the fatigue, or in the case that the fatigue mode switch 60 (refer to Fig. 4) is pushed, the control circuit 30 selectively actuates the seat heaters 36, 38, 40 and 42 arranged at the predetermined positions according to the fatigue so as to generate heat. Accordingly, the shoulder portion, the lumbar portion and the thigh portion are warmed up respectively by the seat heaters 36, 38, 40 and 42.

Further, in the case that the control circuit 30 detects the fluctuation of the breath and the heart rate on the basis of the measured data output from the heart rate sensor 18 and the data corresponding to the body temperature distribution output from the infrared ray sensor 16, and determines that the fatigue of the seated crew is severe, the control circuit 30 actuates the massaging devices 102, 104 and 106 in an interlocking manner. Accordingly, the shoulder portion, the lumbar portion and the thigh portion are massaged respectively by the massaging devices 102, 104 and 106. Therefore, the relaxation generated by the massaging is supplied to the seated crew, as well as the blood circulation of approximately the whole body from the shoulder portion to the lumbar portion and the thigh portion, and to the lower limb end portion by extension is more promoted, and the fatigue of the seated crew is rapidly improved.

As mentioned above, in accordance with the vehicle-use crew heating device 100 in accordance with the fifth exemplary embodiment of the invention, the seated crew is relaxed by the massaging devices 102, 104 and 106, in addition to the effect that the region corresponding to the symptom to be improved of the seated crew is locally warmed up (receives thermal stimulation) by a plurality of seat heaters 36, 38, 40 and 42. Thereby, it is possible to provide a comfortable vehicle interior space for the seated crew.

In this case, in the structure mentioned above, the massaging devices 102, 104 and 106 are incorporated as the relaxation supplying means in the vehicle-use seat 12, however, a low frequency stimulation device capable of applying low frequency stimulation to the seated crew, or a magnetic stimulation device capable of applying magnetic stimulation may be provided in place of the massaging devices 102, 104 and 106.

Further, as shown in Fig. 16, the structure may be made such that an aromatic incense generating device 108 capable of supplying aromatic incense is provided, and the aromatic incense is generated from the aromatic incense generating device 108 working with the operation of the air conditioning device 72 (refer to the description of the second exemplary embodiment with regard to this operation) so as to supply to the seated crew. In accordance with this structure, it is possible to supply the more comfortable vehicle interior space to the seated crew.

### INDUSTRIAL APPLICABILITY

The invention can improve the various symptoms of the crew, and can be preferably equipped in the vehicle such as the passenger motor vehicle or the like.

### Explanation of Reference Numerals

10, 70, 80, 90, 10 vehicle-use crew heating device
12 vehicle-use seat
14 camera (one part of crew state detecting means)
16 infrared ray sensor (one part of crew state detecting means)
18 heart rate sensor (one part of crew state detecting means)
20, 84 blood circulation sensor (one part of crew state detecting means)
22, 24 pressure sensitive sensor (one part of crew state detecting means)
26 pedal operation sensor (one part of crew state detecting means)
28 operation panel (operation intent detecting means)
30 control circuit (control means)
32 seat cushion
34 seat back
36 seat heater (heating element, first heating element)
38, 40 seat heater (heating element, second heating element)
42 seat heater (heating element, third heating element)
52 coldness mode switch
54 stiff shoulder mode switch
56 lumbago mode switch
58 swelling foot mode switch
60 fatigue mode switch
72 air conditioning unit (crew-sensed-temperature adjusting means)
82 steering wheel heater (crew-sensed-temperature adjusting means) 92,94 accessorily heating heater (crew-sensed-temperature adjusting means) 102, 104, 106 massaging device (relaxation supplying means)
108 aromatic incense generating device (relaxation supplying means)

## Claims

1. A vehicle-use crew heating device, wherein a plurality of heating elements are divisionally arranged at least one of a seat cushion and a seat back of a vehicle-use seat according to a plurality of symptoms to be improved of a seated crew, the plurality of heating elements being selectively operated according to a symptom to be improved of the seated crew.

2. The vehicle-use crew heating device of claim 1, wherein the plurality of heating elements include:
a first heating element provided in the seat back at a region supporting a shoulder portion of the seated crew;
a second heating element provided in the seat back at a region supporting a lumbar portion of the seated crew; and
a third heating element provided in the seat cushion at a region supporting a thigh portion of the seated crew.

3. The vehicle-use crew heating device of claim 1 or 2, wherein the crew heating device comprises:
a crew state detecting means for detecting a state of a seated crew; and
control means for selectively actuating the plurality of heating elements based on the detection results of the crew state detecting means.

4. The vehicle-use crew heating device of any one of claims 1 to 3, wherein the crew heating device comprises:
an operation intent detecting means for detecting an operation intent of the seated crew; and
a control means for selectively actuating the plurality heating elements based on the detection results of the operation intent detecting means.

5. The vehicle-use crew heating device of any one of claims 1 to 4, wherein the crew heating device comprises:
a crew-sensed-temperature adjusting means capable of adjusting a temperature which the crew senses; and
a control means for actuating the crew-sensed-temperature adjusting means the plurality of heating elements in an interlocking manner.

6. The vehicle-use crew heating device of any one of claims 1 to 5, wherein each of the plurality of heating elements is configured such that a temperature of a center portion becomes higher than a temperature of a peripheral portion thereof.

7. The vehicle-use crew heating device of any one of claims 1 to 6, wherein the crew heating device comprises:
a relaxation supplying means comprising any one of a massaging device capable of massaging the seated crew, a low frequency stimulation device capable of applying low frequency stimulation to the seated crew, a magnetic stimulation device capable of applying magnetic stimulation, or an aromatic incense generating device capable of supplying aromatic incense to the seated crew, or a combination thereof; and
a control means actuating the relaxation supplying means the plurality of heating elements in an interlocking manner.
